(19) Europäisches Patentamt
European Patent Office
Office européen des brevets

(11) **EP 2 261 857 A1**

(12) **EUROPEAN PATENT APPLICATION**

(43) Date of publication:
**15.12.2010 Bulletin 2010/50**

(51) Int Cl.:
**G06T 7/00** *(2006.01)*     *G06T 7/20* *(2006.01)*

(21) Application number: **09290444.0**

(22) Date of filing: **12.06.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(71) Applicant: **Star Nav**
**14370 Chicheboville (FR)**

(72) Inventors:
• **Ponzo, Jean-Michel**
  **14370 Mery Corbon (FR)**

• **Petegnief, Sébastien**
  **14000 Caen (FR)**
• **Lamy au Rousseau, Georges**
  **14370 Chicheboville (FR)**

(74) Representative: **Grünecker, Kinkeldey,**
  **Stockmair & Schwanhäusser**
  **Anwaltssozietät**
  **Leopoldstrasse 4**
  **80802 München (DE)**

(54) **Method for determining the position of an object in an image, for determining an attitude of a persons face and method for controlling an input device based on the detection of attitude or eye gaze**

(57)     The invention relates to a method for determining the position of an object in an image comprising the steps of: a)determining a first apparent position of the object in an image using a first detection method, b) determining a second apparent position of the object in an image using a second detection method and c) determining the difference between the first and second apparent position, and finally deciding that the real position is based on the first apparent position in case the difference is less than a predetermined difference value and deciding that the real position is based on the second apparent position in case the difference is larger than the predetermined difference value. Furthermore the invention relates to a method of determining an eye gaze direction and corresponding eye gaze detecting device, wherein, based on the determination of additional characteristic facial points in addition to the positioning of the eyes, in particular the irises, the eye gaze direction can be determined with only one image taken from only one camera. The invention also relates to an input device based on the detection of moving direction sequences.

FIG. 1a

**Description**

The invention relates to a method of determining the Position of an Object in an Image, for determining an attitude of a persons face and/or the eye gaze direction of a person, a corresponding attitude and/or eye gaze detecting device and the method for controlling an input device, in particular a switch, based on the detection of attitude or eye gaze of a person as well as a corresponding input device.

There is a need to provide methods that can rapidly and reliably determine the position of an object in image, in particular in case one is interested in the trajectory of the object in a sequence of images. Another important parameter is the stability of the method over time, to prevent unnecessary recalibrations in case a drift occurs. Depending of the application it is furthermore of interest to provide a method that keeps the computational power low, so that the method could be implemented in simple electronic, e.g. hand held devices.

None of the techniques known in the art satisfies all requirements at the same time. Certainly methods like the so called "optical flow" methods, which can follow the position of an object based on the invariance of the colour of a point in image with respect to time, provide a precise localisation of the object, however, are not stable enough over time. An alternative method, which can provide the position of a person's face in an image, is based on the detection of connected pixels having a colour scheme corresponding to a human face, provide a rather stable positioning over time, however, lacks precision.

It is therefore a first object of the invention to provide a method for determining the position of an object in an image that overcomes the problems in the art and that allows without extensive computational power to determine in nearly real-time the position of an object in an image in a precise and stable way.

Furthermore, it is not only the position of an object, in particular the face of a person that is of interest, but also the attitude of a persons head or even the direction the person is looking at, thus the eye gaze direction. In fact, several techniques are known in the prior art to determine the eye gaze direction of a person.

A first method relates to an image data treatment method. A camera films the face of a user and image data treatment algorithms identify the position of the eyes of the user and determine the eye gaze direction. The images can be acquired with one camera, however, a computer with a high calculation power is necessary to carry out the data treatment. Furthermore, with this kind of approach, either the computational power necessary to obtain an accurate determination of the eye gaze is necessary which either makes the process slow or the device used expensive or only a rather low precision of the eye gaze direction is obtained which in addition is not stable in time so that after a certain time the derivation of the direction determination is so large that the process can no longer be used. To improve the precision, it had been proposed to use two cameras to obtain stereographic data to determine a 3D model from which the eye-gaze information is obtained. This approach also needs important calculation power in addition to an additional second camera.

A second method illuminates the eye of a user with infrared light. This technology is based on the fact that two types of reflections occur, namely fixed reflections of the infrared light coming from the cornea and mobile reflections coming from the pupil. Using the different positions between the two families in the taken images the eye gaze direction can be determined. This method, even though facilitating the data analysis and being more precise than the first method, has the negative drawback that specialized equipment, namely an additional infrared light source and a corresponding camera, is necessary.

A third method is based on the use of magnetic contact lenses used by the user. Each movement of the eye causes a change in the external magnetic field which can be detected by corresponding sensors. This method is very precise, however, the necessary equipment is expensive and users might refrain from carrying the magnetic contact lenses fearing health consequences.

Even though each one of the known methods provides possibilities to determine automatically the eye gaze of a person, there is a need for simpler methods which can be carried out with less demanding equipment but nevertheless provide precision and robustness. It is therefore the second object of the present invention to provide an improved method for detecting the attitude of a persons face and/or the eye gaze of a person which can in particular be carried out using only one camera.

In addition, input devices exist that use the detection of eye gaze of a person to provide an input to a device, e.g. a computer or a machine, without having to use the hands. Such an input device has to provide a rapid and reliable response to an input. It is of special importance to be able to provide a robust decision whether an input is intentional or unintentional.

It is therefore a third object of the present invention to provide a method for controlling an input device using eye gaze which is rapid and reliable.

The first object of the invention is achieved, with the method according to claim 1. It relates to a method for determining the position of an object in an image comprising the steps of: a) determining a first apparent position of the object in an image using a first detection method, b) determining a second apparent position of the object in an image using a second detection method, c) determining the difference between the first and second apparent position, d) deciding that the real position is based on the first apparent position in case the difference is less than a predetermined difference value and deciding that the real position is based on the second apparent position in case the difference is larger than the predetermined difference value. By applying this inventive method, one can take advantage of the distinct advantages of the first and second detection methods and therefore in sum achieve improved results, e.g. with respect to speed, reliability and reduced computational power.

Preferably, the object can be a part of a living body, in particular a face or hand of a person. Following a person's face or hand in an image can be of interest in numerous applications, e.g. as part of a process of determining a person's attitude or even eye gaze.

Advantageously, the steps a) to d) can be repeated for a sequence of subsequent images. The inventive method is thus not only limit to the analysis of a single image, but the change of position of the object, e.g. a person's head can be followed in time. Due to the use of at least two different technologies, the process can be carried out real time with surprising stability in time without excessive computational power.

According to a preferred embodiment, the first detection method can provide the position with a lower error than the second detection method and/or the second detection method can have a lower drift than the first detection method. Over short time frames it is thus the first method that will provide the desired result, whereas drift of the result that might occur in the precise method will then be taken care of by the second process.

Preferably, the position of the object will still be determined by the first detection method, but the result of the second detection method is used to recalibrate the first detection method. Thus the position of the object is always determined using the same algorithm, in particular the one with the reduced error on the measurement.

Further preferred, the first detection method can be an optical flow method, in particular a Lucas-Kanade method, a block matching method or a Mean Shift method. The optical flow method, which is based on the follow-up of the object in subsequent images based on the invariance of colour of one point of the image with respect to time. This method provides the desired precision concerning the position of the object. Over time this method, however, looses its calibration and the origin of the position determination fades away and becomes erroneous.

Preferably, the second detection method can be a face detect method, in particular based on a Haar algorithm, a segmentation algorithm or pixel colour based. This kind of algorithm provides the desired stability over time, but suffers from a lack of precision. Thus in combination with the optical flow methods all desired parameters can be controlled.

According to a preferred embodiment, the predetermined difference value is between 20 to 40 pixels, in particular 30 pixels. Below this value, the recalibration occurs too often, whereas for higher values the position is deviating too much, to be used for practical applications.

The described method can preferably be used for controlling an input device. By using positional information the information provided by the method described an input device, e.g. an electronic device, like a computer, or a switch, can be controlled without having to touch the device. Thus the method can be used as an input providing method especially adapted to handicapped people who for instance might no be able to use their hands to control devices. By moving their head/face it becomes possible to indicate an input to the device.

According to a preferred realization, the position of a cursor on a screen can be controlled using this method. In this case, having identified a direction and a distance corresponding to the movement of the head of a person or its hand, the cursor on the screen can be moved in the same or at least proportional way.

Advantageously, a change of position of the cursor from one image to the next can be determined by the positional change of the object from the one image to the next image. With the inventive method using two different methods to determine the position and subsequently a change in position, a real time cursor control becomes possible.

The second object of the invention is achieved with the method of determining an attitude or eye gaze direction of a person according to claim 10 and comprising the steps of: a) receiving image data of the person's face from a camera, the image data corresponding to the data of one, in particular only one, image of the person's head, b) determining the position of at least three characteristic facial points in particular positioned away from the eyes of the person, and c) determining the angular position of the face with respect to a reference system of the camera based on the positions of the characteristic facial points to thereby determine the attitude of the person's face.

With the method according to the invention, it is possible to determine the attitude of a person face in a simple manner only using the image data of one, in particular only image taken of the person and without needing an additional light source, like an IR light source like in the prior art. It is the surprising founding of the invention that by taking into account only some characteristic facial points, as for example two points situated in the periphery of the eye and a third point associated with the nose, the attitude of a person's face in the reference system of the camera can be obtained. The use of only some, for instance three are enough, characteristic facial points has the advantage that the method can be carried out with hardware having relatively low computational power and thus the method can be realized with cheap devices. Nevertheless the attitude can be determined in a robust way. Based on the characteristic facial points, it becomes indeed possible to establish a link between the postures of the person's head with respect to the camera. The data is used to determine the attitude, thus the angular position of the face with respect to the plane of the camera, thus a fixed reference system. With this analysis one can take into account whether the person's face is inclined to the left or the right, to the front or the back or turned to the left or the right.

Preferably, only one camera can capture the image, in particular from a fixed position independent of the person. Thus, unlike the in prior art which uses two cameras to be able to establish a 3D model, the inventive method can be carried out with only one camera. In fact, using the characteristic facial points a virtual three dimensional model can be established. Furthermore the camera does not have to be attached to the user's head which simplifies the use.

Advantageously, the method can furthermore comprise an initialisation step of determining the distance between the person and a predetermined plane, e.g. the plane of the camera. Knowing this distance and knowing the position of the characteristic facial points the attitude in the reference system of the camera can be determined with sufficient precision, in particular in cases when the distance between the person and the camera remains constant.

This step can be carried out upon powering up of the device carrying the invention, in particular upon the detection of a face in an image supplied to the device. Eventually the distance determining step can also be repeated, e.g. on a regular basis, to take into account changes in the distance between the user and the camera.

Preferably, the distance between the person and the camera can be determined based on the real distance between two fixed facial points and their distance in the image. Indeed, knowing the characteristics of the camera used (focal distance, number of pixels ...), and the distance separating two points of a person in the image and the real world, the distance between the user and a camera is easily and rapidly established.

Preferably, the two facial points are the eyes of the person. Due to the particularity of the eyes (circle in a white background), these two points can easily be determined out of the image and, as a consequence, their distance in the image be rapidly and precisely determined.

Advantageously, the real distance between the facial points, in particular the eyes, can be received manually from a user input or corresponds to a fixed value, in particular to a value between 56 - 76 mm, more in particular $65 \pm 3$ mm. Thus, it is possible to either provide the exact value, improving the resolution but needing an active input by the user or to use a standard value of about 65 mm which corresponds to the average distance between the eyes in the case of an adult person, in which case the initialisation can be carried out automatically.

Preferably, during the initialisation, the user can be requested, in particular visually and/or acoustically, to look straight right into the camera. In this case, the distance between the two fixed facial points determined is maximal, and by comparison with the value of the real distance between these two points, the distance to the user is established.

According to a preferred embodiment, the method can further comprise a step of determining the facial portion of the person in the image prior to step b). By determining the part of the image that comprises the face, the amount of data to be treated later on to determine the position of the characteristic facial points and the position of the eyes is simplified due to a reduced amount of data. The identification of a face in an image can, for example, be based on the colour characteristics of the skin using, for instance, a neural network.

Preferably, the method can be extended to determine the eye gaze direction, wherein step b) comprises an additional sub-step of determining the position of at least one eye, in particular the iris of the eye, wherein the characteristic facial points are positioned away from the eye, and further comprises a step d) of determining the eye-gaze direction in the reference system of the camera based on the angular position of the face and the position of the at least one eye, in particular of the position of the iris and the position of the eyeball centre of the eye. Taking into account both the position of the eye, in particular the position of the iris, in the image taken, the eye gaze direction is established using the angular position of the face with respect to the fixed reference system of the camera. Thus based on a two step process, determining the position of the eyes on the one side and determining the attitude based on the characteristic facial points, the eye gaze direction in the reference system of the camera is determined in a simple but reliable manner. On the same time, only one camera is necessary.

Preferably step d) of the method for determining the eye gaze direction, can comprise taking into account the coordinates of at least one characteristic facial point or the coordinates of the eyes in the image to determine a bound vector of the eye gaze direction relative to the position of the camera. Thus not only a direction but also the position where a user is looking at can be determined. Thus, for instance in case the user looks at a screen and knowing the relative position between camera and screen, the method provides a simple but reliable way of determining the exact position on the screen the person is looking at.

According to an advantageous variant, the method can comprise determining the eye gaze direction in a plurality of images, wherein in the first image the eye gaze direction is determined as described above, and wherein in the subsequent images the eye gaze direction is determined based on relative changes of the position or again in absolute terms.

The first objects of the invention are also achieved with an attitude or eye gaze detecting device comprising one camera and a processing unit configured to carry out at least one of the methods with the features as described above. With this eye gaze detecting device, all the advantageous effects of the method described above can be achieved with a minimum amount of hardware components necessary. For instance, the computational power of a standard mini PC, based on an Atom 170 processor is suitable to carry out the method of the invention.

The objects of the invention are also achieved with a computer program product, comprising one or more computer-readable media having computer executable instructions for performing the steps which can be executed by the computer of the method as described above.

The third object of the invention is achieved with a method for controlling an input device, in particular a switch, based on the detection of the position of a person's head, in particular its eye gaze, comprising the steps of: a) receiving image data corresponding to the data of a plurality of images taken of at least a part of the person's face; b) determining a sequence of moving directions of the person's head, in particular its eye gaze; and c) identifying an input to the device, when the determined moving directions correspond to a predetermined sequence of moving directions.

The method for controlling an input device according to the invention takes advantage of identifying a moving direction of the eye gaze which has the advantage compared to methods that request a user to look at a given point for a predetermined time, that the moving direction of eye gaze can be obtained by simply comparing a plurality of subsequent images and no absolute determination is necessary. This simplifies the data processing and still provides a robust suppression of unwanted inputs. In this context, the term moving direction of the eye gaze relates to the direction along which the eye gaze evolves over a predetermined amount of subsequent images. It therefore does not relate to the eye gaze direction itself.

Preferably, the sequence can comprise at least one moving direction, in particular more than one moving direction. The use of more than one moving directions of the eye gaze has the advantage that one can effectively suppress unintentional inputs to the device, as the probability for an unintentional matching is low. For instance, a moving sequence could comprise: a) looking up; b) looking to the left; and c) looking to the right.

In particular, the use of at least one direction being perpendicular to the other ones is suitable to exclude unintentional inputs.

Advantageously, the moving direction in step b) can be determined by determining the eye gaze in one image according to the methods as described above. As explained above, these methods can be realized using only one camera and low computing power is sufficient to establish the eye gaze direction in a simple but reliable way.

Advantageously, the method can furthermore comprise the step of outputting an indication to the user of the next moving direction of the eye gaze in the sequence. Preferably, the indication can be a visual indication, in particular using a light source and/or a display, and/or an audio indication. By outputting an indication about the next moving direction in the sequence, the user can be guided so that an input can be realized without that the user has to know beforehand the exact sequence needed to instruct an input. This further simplifies the method of inputting instructions.

Preferably, the method can furthermore comprise a step of confirming the input comprising identifying the matching of the eye gaze direction of the person with a predetermined direction in a plurality of subsequent images and/or the identification of a predetermined eye blinking sequence, for instance a double blink.

Advantageously, a plurality of different predetermined sequences of moving directions can be provided wherein each predetermined sequence is attributed to a particular input, to thereby provide a plurality of different input signals to the device. Thus with the method not only binary switches can be controlled, but also device with multiple outputs can be controlled. This method could thus be used to input numbers or letters depending on the sequence to control an electronic device, like the switching to a particular TV station or the inputting of a certain floor in a control unit for an elevator.

The invention also relates to a computer program product, comprising one or more computer-readable media and computer executable instructions for performing the steps of the method as described above.

The third object of the invention is also achieved with an input device which comprises one camera and a processing unit configured to carry out the method for controlling an input device described above. This input device can, for instance, be a switch. With this input device the advantageous effects as described above can be achieved.

Advantageously, the input device can furthermore comprise a plurality of light emitting elements arranged according to the sequence of moving directions relative to a central position and/or a display. The light emitting elements and/or the display can be used to indicate to the user of the input device about the change in eye gaze direction that has to be made to input an instruction to the device. According to a further variant, the visual indication could be replaced or accompanied by an audio source configured to output the corresponding instructions, for instance "looking left", "looking right", "looking up" or "looking down".

Preferably, the camera can be positioned in a central position with respect to the light emitting elements with one first light emitting element being positioned to the left, a second light emitting element being positioned to the right and the third light emitting element being positioned above or below the central position. Eventually a fourth light emitting element could be positioned below or above the central position complementary with respect to the centre to the third light emitting one. Using these light emitting elements, the eye gaze of a user can be directed up and down or left and right depending of the predetermined sequence a user has to carry out to provide the necessary input.

Further features and advantages of the invention will be described in the following practical embodiments of the invention and with respect to the subsequent Figures:

Figures 1a to 1c     schematically illustrate a method to determine the position of an object in an image according to a first embodiment of the invention,

Figure 2          schematically illustrates a flowchart of a method of determining an eye gaze direction according to a second embodiment of the present invention,

Figure 3          illustrates an input device according to the invention,

Figure 4          illustrates a typical image taken by the camera of a person the eye gaze direction of whom will be analyzed,

Figure 5          illustrates schematically the algorithm used to determine the eye gaze direction according to the invention,

Figures 6A to 6D     illustrate a third embodiment according to the invention, namely a method for controlling an input device, in particular a switch, based on the detection of eye gaze of a person, and

Figures 7A to 7C     illustrate three inventive applications of the eye gaze determining methods according to the first to third embodiments and the corresponding eye gaze detecting device and the input device.

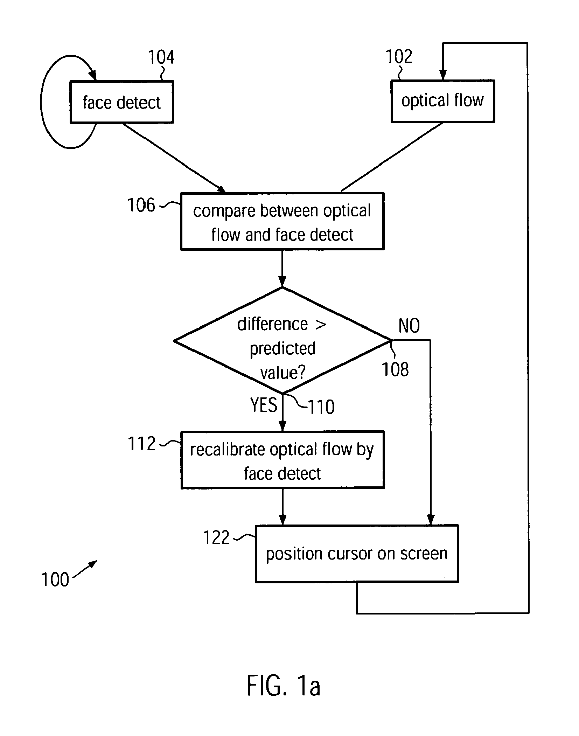

Figure 1a illustrates schematically a first embodiment of the invention to determine the position of an object in an image. In this embodiment, the method is used to determine the positional change of a person's head in a series of subsequent images, for example taken by a video camera, in particular a web cam. The result of this analysis is then provided to an input device of an electronic device, like a computer to control the movement of the cursor on the screen of the computer according to the change in position of the person's face. Instead of a person's face it is also possible according to the invention to follow any part of the body like a hand, etc.

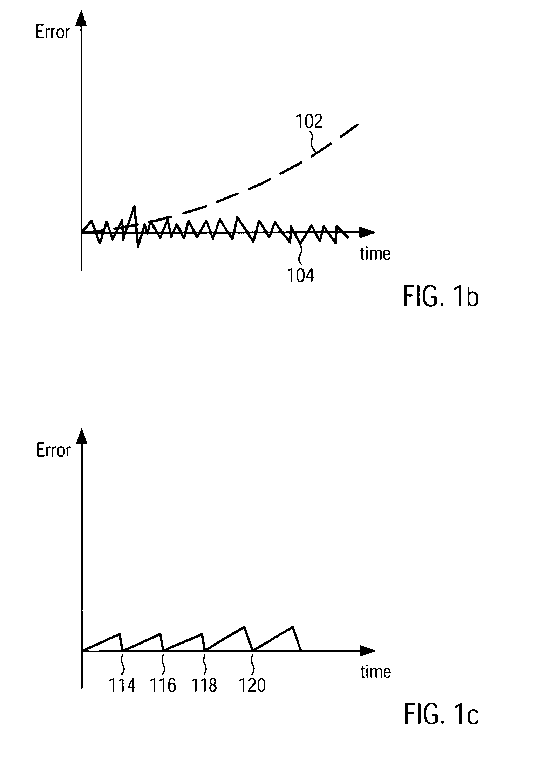

The method illustrated in Figure 1 a has the particularity to provide two different algorithms to identify the position of the object, here thus the user's head, at the same time.

On the one hand the method 100 according to the first embodiment uses a so called optical flow method 102, in particular using a Lucas-Kanade method, a block matching method or a Mean Shift method to determine the apparent position of the object in an image, thus the person's face in an image. Here the positioning is actually based on the motion from one image to the next. The algorithm is based on the follow up of an object in a sequence of images and takes advantage of the invariance of the colour of a point in the image with respect to time. For instance one can follow the edge region of the eye bows of a person from one image to the other. With this method a very precise positioning can be achieved without a too heavy data treatment necessary. However, the drawback of this kind of algorithm is its tendency to a large drift in time. For instance, in case a second person appears in the image, the process gets confused and the position of the point one wants to follow is no longer guaranteed.

To improve the method it is therefore proposed to use a second method to determine essentially at the same time also a second apparent position of the object in the image using a second detection method 104. The position determination is preferably repeated for each image to be analyse. In this embodiment the second detection method 104 consists in using a so called face detect method, in particular based on a Haar algorithm, a segmentation algorithm or pixel colour based. This type of method allows detecting the human face in an image and outputs a position typically based on the barycentre. This kind of analysis typically does not show a drifting origin, however the error on the position is relatively large compared to the first detection method. It is therefore not used to precisely determine a person's face position in an image.

Figure 1b illustrates the properties of the two different methods used to determine the position of the user's face in an image. Whereas the first method 102 determines the position of the object in a rather precise manner within a rather short time frame, it has a tendency to drift away at longer time intervals. In contrast thereto the second method 104 determines the object's position with a large error bar, however it does not drift away over time.

The invention according to the first embodiment, takes advantage of the good properties of the two different algorithms and by combining them it becomes possible to reject the negative ones.

To do so the next step 106 consists in comparing the position obtained by optical flow 102 and the one of face detect 104. Then in case the difference is less than a predetermined tolerance level 108, the process continues by using the apparent position determined by the first detection method 102 to declare the position determined by the first detection method 102 to be the real position of the object, thus the user's face, in the image. If, however, the difference is larger than a predetermined tolerance level 110, the first detection method 102 is recalibrated 112 using the position of the second method 104. Once recalibrated the first method is nevertheless used to determine the position in the image.

The concept of steps 106 to 112 is illustrated in Figure 1c. Once the difference in position between the first and second method becomes too large, e.g. at position 114, the method decides to recalibrate the origin of the first detection method and then again uses the results of this method to continue the process. The same occurs at time positions 116, 118 and 120.

Thus, using two different techniques to determine the position of the object, here the person's face, in an image, it becomes possible to optimize precision, reliability, stability in time and reduces computational power. In addition, the method can be carried out in real time using a simple computer, e.g. using Intel's Atom processor.

One possible application of the method according to the first embodiment is illustrated in Step 122. Here the result of the position in the image is used to position the cursor on the screen of a computer.

The method is then repeated again, upon reception of a new image and based on the change of the position, the cursor can be moved over the screen.

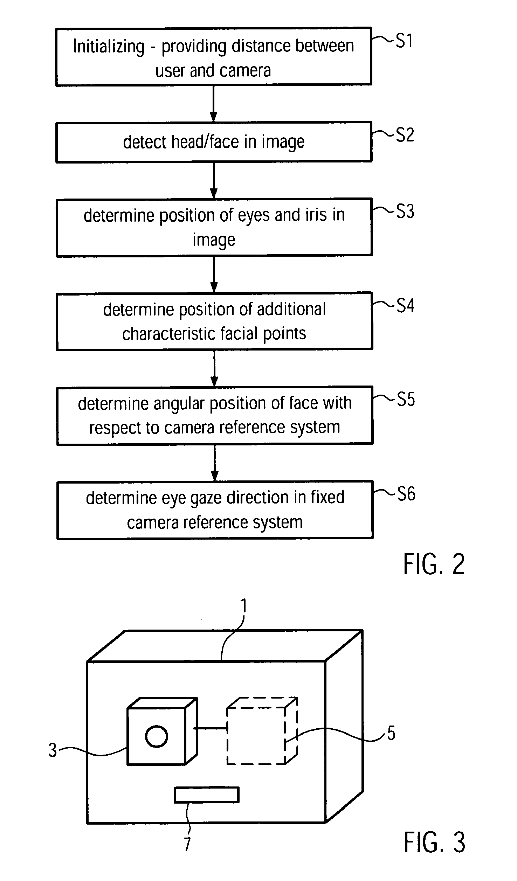

This method can advantageously be used for handicapped persons as it allows them to command a computer without needing their hands. Simply a move of the head allows to move the cursor. As the process can be implemented on a rather simple computer 5 and using a digital camera 3 like illustrated in Figure 3, a less expensive device than already present in the art can be offered.

Figure 2 is a flowchart of a method of determining an attitude, or even the eye gaze direction according to a second embodiment of the present invention. The method illustrated in Figure 2 can be carried out with a rather simple eye gaze detecting device according to the invention and which is illustrated in Figure 3.

The eye gaze detecting device 1 comprises a camera 3 and a processing unit 5. The camera 3, typically a digital photo or a video camera, is used to capture images of a user's head and the processing unit is configured to analyse the image data received from the camera 3 to determine the eye gaze direction. Using the method illustrated in Figure 2, the eye gaze direction can be determined without needing any further additional equipment like, for example, an additional light emitting device, in particular an infrared light emitting device as used in some methods of the prior art, or an additional camera to be able to obtain stereographic data information of the person to set up a 3D model.

Upon starting up of device 1, an initialisation step S1 is carried out which is used to calibrate the system to obtain parameters necessary to establish the eye gaze direction determination process carried out by the processing unit 5.

In this embodiment, the initialization step S1 comprises estimating the distance of a user with respect to the eye gaze detecting device 1. This information is needed when, based on the eye gaze direction, one would like to determine the coordinates of a point a person looks at. This point can be in the plane of the camera 3, but could also be linked to a display. In this case the distance between the user and the display needs to be determined. In case the camera 3 and the display have a fixed relationship, it is again sufficient to determine the distance between user and camera.

The distance between the user and the eye gaze detecting device 1 can be carried out according to a plurality of different methods.

According to one possibility according to the invention, a user can manually enter the distance after, for example, having measured his distance from the device.

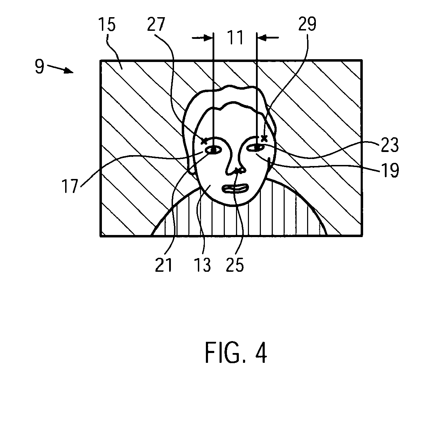

Preferably, the distance determining step is, nevertheless, carried out automatically. To do so, advantage is taken of an image taken by the camera 3. Figure 4 illustrates a typical image 9 taken by the camera 3 of a person. In image 9, reference numeral 11 indicates the inter-pupil distance which can, for example, be expressed in a number of pixels. Indeed, it is possible to estimate the distance between the user and the device 1 by establishing the angle separating two predetermined facial points, in particular the distance between the two eyes using for instance the distance between the pupils. To do so, the processing unit 5 is configured to identify the eyes in the image and to determine their distance in the image taken. Then, knowing the characteristics of the camera (focal position, number of pixels) and the real distance between the eyes or an estimate thereof using the properties of an average person - the inter-pupil distance of an average person is 65 mm - the distance separating the user from the camera 3 can be obtained without any manual input from the user. Eventually, to adapt the eye gaze detecting device 1 to a particular user, the user might manually input his own inter-pupil distance, measured by hand, for example. This value could be stored in the device so that the manual input is only needed for the first use.

The initialisation procedure could be accompanied by providing an indication to the user to look at a certain predetermined point having a fixed spatial relationship with the camera 3.

According to a further embodiment of the eye detecting device 1 according to the invention, a display can be part of the device 1. A message on display 7 can advise the user to look at a given point, e.g. also shown on the device. The display does not need to be part of the device 1, but can be a display connected to device 1. In addition, at the same time a message could be output, asking the user to hold his head in a straight posture. In this case it can ensured that the distance between the eyes in the image is maximal, if the user looks into the camera under an angle the distance observed in the image will reduce which would create an error in the distance determination.

According to a further variant, during initialisation the user can be invited to look at particular additional points on display 7. This additional data can then be used in case a higher resolution is necessary.

According to an additional variant of the second embodiment, the step of determining the distance between the device and the user could be calculated on regular basis to take into account any movements of the user.

The next step S2 in the method illustrated in Figure 2 relates to the detection of a part of the image showing the head, or preferably the face, of the user. This step is not mandatory to the embodiment according to the invention, and in a variant of the invention according to the second embodiment steps S2 is not carried out, but has the advantage that the amount of image data to be analysed to detect the eye gaze direction is reduced by suppressing the background of the image from the data to be analyzed later on. One method to identify the facial part of the image is based on the use of a neural network and re-grouping those parts of the image that show skin colour in a connected area of the image. Indeed, like illustrated in Figure 4, the colour of the skin can be used to discriminate the facial part 13 of the image 9 from the background region 15. Nevertheless, other suitable methods to detect the facial portion of the image, such as shape recognition methods based on statistical training or colour based image segmentation, could be applied.

The next step S3 (see Figure 2) of the method according to the invention consists in determining the positions of the eyes 17, 19 of the user (see Figure 4). A typical method to determine the position of the eyes is based on the analysis of contours. As white colour areas are present in the eye region, the contrasts around the iris are easy to detect (derivative methods). To detect the circles of the irises 21, 23, the Hough algorithm can be used. Barycentre determination is another method. It gives an important advantage to detect ellipses instead of circles, especially when the user's face rotates relatively to the camera. The method used are adaptations of the one used for the circles. Another method is based on pattern recognition, e.g. creating an eye model and researching the position on the image where the model fits at best or acquiring an image and researching from one image to the following the former acquired pattern.

Nevertheless, any other suitable method to detect the position of the eye can be applied.

It is the special characteristic of this invention that, according to step S4, not only the position of the eyes 17, 19 in image 9 is determined, but that additional characteristic facial points are analysed to determine the eye gaze direction. In this embodiment, three additional facial points are identified in the image, however, more than three additional characteristic facial points could be analysed, in case the resolution has to be improved.

In image 9 illustrated in Figure 4, the characteristic facial points are the lower tip of the nose 25 and points 27 and 29 in the peripheral region of the eyes 17, 19, here in particular, the outer ends of the eye brows. Other suitable facial points could be the ears (if visible), the corners of the mouth, beauty patches etc.. Eventually, according to a variant, a detailed analysis of the image 9 of the person taken during initialisation could be carried out to identify particular facial points in the face of the user.

Based on the positions of the additional characteristic facial points and the additional parameters determined during initialization, the processing unit 5 then determines the angular position of the head of the user with respect to the fixed reference system of the camera, which is also called the attitude (Step S5).

From the position of the additional characteristic facial points 25, 27, 29, the processing unit 5 is thus able to determine the inclination up or down, a turn to the left or to the right and an inclination to the left and right of the head. These movements have an impact on the eye gaze direction. Based on the positions of the three additional facial points, furthermore translational movements of the head which have an impact on the exact point a person is looking at, can also be determined, movement to the left and right and movement forward and backward.

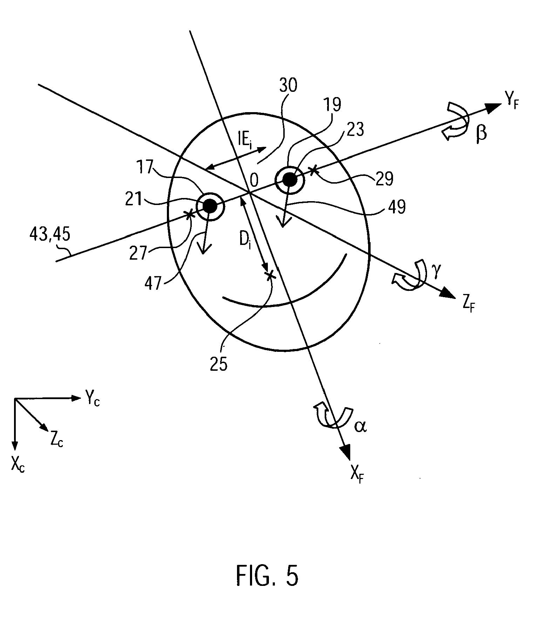

Having determined the attitude of the user's head in the camera's reference system 41 illustrated in Figure 5 illustrating the position of the eyes 17, 19, the position of the iris 23, as well as the additional characteristic facial points 25, 27 and 29, a reference system 43, 45 attached to the eyes 17, 19 is obtained. Knowing the relationship between the two reference systems: fixed reference system 41 of the camera and reference system attached to the user 43, 45, the absolute eye gaze direction 47, 49 in the fixed reference system 41 of the camera 3 is obtained based on the transformation between the reference systems 43, 45 attached to the eye and the details about the positions of the irises 21, 23 (Step S6).

In one practical example according to the invention, a first distance IE (inter eye) is determined between the two higher facial points 27 and 29. In the same way, the second distance $D_i$ between the middle of the segment relying the higher facial points 27 and 29 (point 30 on Figure 2) and the third facial point 25 is determined out of the image. The maximum values for IE, thus $IE_{max}$ and for D can be obtained out of an image during initialization during which the user was looking straight into the camera.

Based on the following equations the angles $\alpha, \beta, \gamma$ illustrated in Figure 5 and defining the attitude of the users face can be obtained as follows:

$$\sin(\gamma) = \frac{X_{Ri} - X_{Li}}{IE_i} \quad \cos(\alpha) = \frac{IE_i}{IE_{MAX}} \quad \text{and} \quad \cos(\beta) = \frac{D_i}{D_{MAX}}$$

in these equations $X_R$ corresponds to the abscise of facial point 27 in the reference system of the camera $X_L$ to the abscise of facial point 29.

Those angles $\alpha, \beta, \gamma$ are thus an estimation of the face's roll, pitch and yaw angles with respect to the reference system of the camera. The measurement of those quantities allows estimating the position of eyeballs 17 and 19 also based on the distance of the user from the camera established during the initialization step by for instance taking into account the distance between the two eyes which is about 65±3 mm.

Then the centre of the eyeballs 17 and 19 can be obtained. To facility the calculations one can make the approximation that the orbits are spherical. Then the position of the irises is detected and a vector joining the eyeball centre and the iris 21, 23 centres can be determined. The final result is the intersection of the vector's 47, 49 direction with the camera plane. Translational movements of the user parallel to the camera plane can be taken into account by looking at changes in the coordinates when the distance IE and D do not change.

Thus, taking into account not only the fixed reference system 41, like in the prior art image analyzing processes using the image of only one camera But also the reference system 43. 45 attached to the attitude of the person's head, the absolute eye gaze direction 47, 49 in the fixed reference system can be determined by only using the coordinates of the five facial points. Thus, compared to other methods using image analyses of more than one image, to obtain a 3D stereoscopic analysis, or using additional light sources such as infrared, the method according to the invention is greatly simplified. In fact, based on the five facial points, a virtual 3D modelling is carried out to obtain the desired absolute eye gaze direction 47, 49 in the fixed reference system. As only a limited number of points has to be analysed, the eye gaze direction can be determined in real time using a standard camera, e.g. with 640 x 480 pixels And a simple processing unit like a Atom 170 mono core processor for embedded systems. Essentially, the inventive method enables a process with which the eye gaze direction can be determined with a device 1 being simpler than the known systems but with which the same results can be obtained.

The eye gaze detecting device 1 can for instance be used to activate certain applications of a computer or to wake up a computer out of a power-down mode when the eye gaze detection device 1 recognises that a user looks at the screen. When subsequent images are analysed a change in the eye gaze direction can be used to move an optical indicator like a mouse on a screen. Without departing from the scope of the invention, the method can also be carried out without determining the position of the iris and only the information about users head attitude with respect to the camera eventually together with the position of the head can be used realize the above mentioned applications.

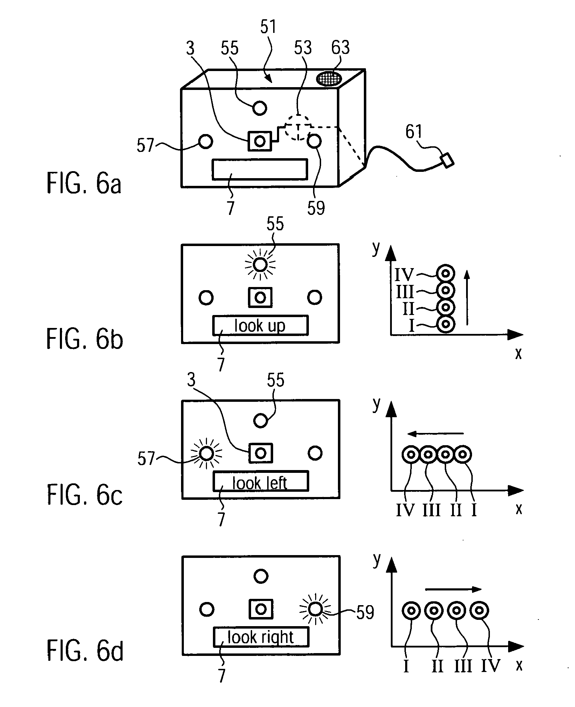

Figures 6a to 6d illustrate a third embodiment according to the invention, namely a method for controlling an input device, in particular a switch, (it can permit to select a floor by eye movements to be defined and to validate it in an elevator) based on the detection of eye gaze of a person. The input device is configured such that upon the detection of a predetermined sequence of moving directions of the eye gaze an input to the device is acknowledged. In the case of a switch this input results in a state change of the switch, thus form on to off or vice versa. Figure 6A furthermore illustrates an embodiment of an input device 51 suitable to carry out the method according to the third embodiment.

Figure 6A illustrates an input device 51 comprising a camera 3, a processing unit 53, a display 7, a first light emitting element 55, such as an LED, a second light emitting element 57, a third light emitting element 59 and an output, here in the form of a connector 61. The processing unit 53 can realize the same functionalities as processing unit 5 of the second embodiment. Elements carrying reference numerals already used in the previous Figures are not repeated again in detail, but their description is incorporated herewith by reference.

The three light emitting elements 55, 57 and 59 are arranged such that the first element 55 is positioned above the camera 3, the second element 57 to the left of the camera 3 and the third element 59 to the right of the camera 3. This arrangement represents only one variant according to the invention as, the three elements could also be arranged differently with respect to the camera 3. Furthermore, more than three light emitting elements could be provided or the display 7 could cover a main part of the surface of device 51 and pixel regions corresponding to the positions of elements 55, 57 and 59 could be used in the same way as the three light emitting elements 55, 57 or 59.

In the method according to the third embodiment, the light emitting elements 55, 57 and 59 play the role of indicating a direction where a user should look at so that, upon accomplishment of a predetermined sequence of eye gaze moving directions, the processing unit 53 of the input device 51 can determine that an input was provided to the device so that, for example, a switching instruction is provided at the interface 61. Instead of using a visual indication using the light emitting elements 55, 57 and 59 or in addition thereto, device 51 might furthermore comprise an audio unit 63, for instance a loud speaker, to provide an audio indication to the user.

In Figures 6B to 6D, one way of carrying out the inventive method using device 51 is illustrated. Figure 6B illustrates the front panel of device 51 after the processing unit 53 has detected a person's face in an image taken by the camera 3.

Upon detection of a face, the processing unit 53 provides a signal to switch on light element 55. In addition or as an alternative, a message is shown on display 7. Here for instance "please look up". An indication is therefore given to the user to look up. The processing unit 53 analyses the eye gaze direction in a series of images taken by camera 3 after having identified a face and derives a moving direction of the eye gaze from that sequence of images. To determine the eye gaze direction in one image, use can be made of the method according to the second embodiment, however, also the method of the first embodiment could be used, in which case one would identify the movement of the face or head of the user to check whether the sequence of predetermined directions has been followed.

According to the invention, the eye gaze direction is not determined in absolute terms but the moving direction of the eye gaze is determined. Thus the processing unit 53 is configured to look at differences in the eye gaze direction between subsequent frames.

In this embodiment, the processing unit 53 is configured such that it determines whether at least the vertical coordinate (y coordinate illustrated in the coordinate system on the right hand side of Figure 6B) of a plurality of subsequent images (indicated by I, II, III, IV) changes in the direction indicated on display 7. In case an increase in the y coordinate is detected over a predetermined amount of image frames, processing unit 53 is configured to decide that the person has effectively looked up.

Upon identification by processing unit 53 that the user has indeed looked up, the processing unit 53 switches off the first light emitting element 55 and switches on the second light emitting element 57 to the left of the camera 3. Alternatively, or in addition, the display 7 indicates a corresponding message namely "please look left". The processing unit 53 again analyses a sequence of images taken by camera 3 and, upon the detection of the moving direction of the eye gaze to the left, thus a change in at least the x coordinate in subsequent images changes in the negative direction of the x axis is determined, concludes that, indeed, the user looked to the left. Again, the moving direction of the eye gaze is determined not in absolute terms but in relative terms.

When the processing unit 53 has identified a predetermined amount of subsequent image frames in which the eye moved horizontally to the left, light emitting element 57 is switched off and light emitting element 59 is turned on as indicated in Figure 6D. At the same time or instead of, the display 7 indicates the message "please look right". The processing unit 53 then again analyses a predetermined amount of image frames (I, II, II, IV) to see whether the eye gaze direction moves to the right, by analyzing the evolution of at least the x coordinates of the eye gaze in subsequent images.

Upon the detection of the predetermined moving direction sequence of the eye gaze: first looking up, second looking left and third looking right, the processing unit identifies a voluntary input of the user and instructs the interface 61 accordingly.

The described predetermined sequence of moving directions of the eye gaze: up, left, right, represents only one example of realising the third embodiment of the invention. By combining several moving directions, in particular with perpendicular directions, a robust decision process discriminating unintentional inputs is provided. Furthermore, by looking at moving direction, thus a relative positioning in subsequent image frames, the analysis can be carried out faster than in case a person has to look in a certain direction, so that the switching process can be carried out in real time.

By furthermore providing the user with indications about the sequence to be carried out, a user-friendly input device based on the detection of eye gaze is provided.

Nevertheless, device 51 according to a variant of the invention can be configured such that more than one input can be provided by the user depending on the sequence of the moving directions. Thus for a given predetermined sequence the device will realize a certain action. For instance a user could choose the floor of an elevator by realizing a first sequence if he wants to reach one floor and a different sequence when he wants to reach another floor.

According to a variant of the third embodiment, the method of the second embodiment could be applied and, for instance, the absolute eye gaze direction detection be used to check whether the person looks precisely at the various points of the light emitting elements.

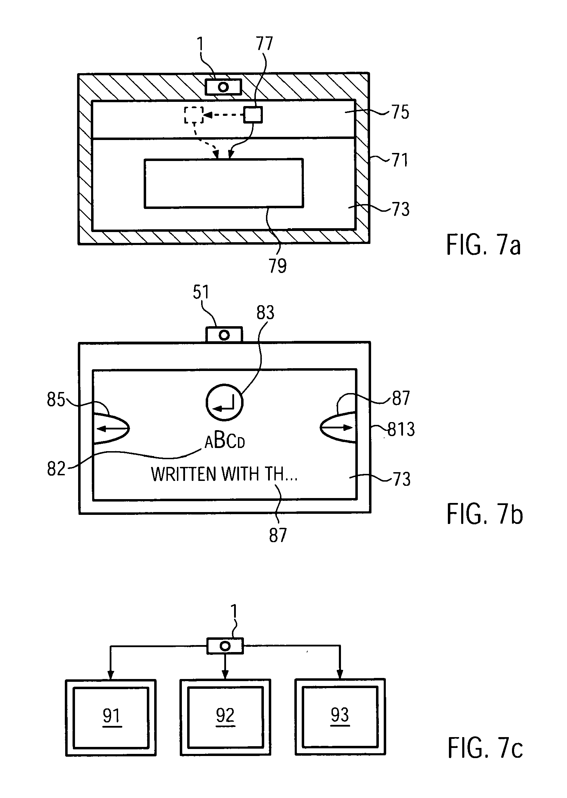

Figures 7A to 7C illustrate three inventive applications of the eye gaze determining methods according to the first and third embodiments and the corresponding eye gaze detecting device 1 and the input device 51.

Figure 7A illustrates a display 71 with an eye gaze detecting device 1 according to the second embodiment incorporated into the display 71. The display 71 is split into two parts, a lower part 73 and an upper part 75. The small box 77 illustrated in the upper part 75 indicates the position a user is looking at, which is determined by the eye gaze detecting device 1. The display 71 is configured such that the area the person is looking at, thus the box 77 is magnified and illustrated in the lower part in box 79. By changing the eye gaze direction within the upper part 75, indicated by the dashed arrow and the dashed box 81, the user can choose to magnify another part of the information displayed in the upper part 75.

This application can be used to choose different applications of an electronic device, like a computer, by looking at one of the icons illustrated in the upper part 75. The application corresponding to the icon a user is looking at is opened up in the lower part 77 of the display 71. According to another inventive use, one can zoom into the detail of an image illustrated in the upper part 75 in its total, whereas the zoomed in part is then shown in the lower part 77. Instead of choosing different icons, it is also possible to change parameters, for instance the volume of a radio or the temperature of an air-conditioning in a car without having to use the hands just by looking in a specific area on a screen.

Figure 7B illustrates a second application showing a screen 81 and an input device according to the third embodiment 51, which in this embodiment is not directly incorporated into the display 81, but according to a variant, could also be included as shown in Figure 7A. The application illustrated in Figure 7B allows a user to input text into an electronic device, such as a computer. In the middle 82 of the display, the characters of the alphabet are illustrated. The largest letter is the one a user can choose by looking at the enter symbol 83. To change the letter, the processing unit 53 of the input device 51 is configured such that, upon the detection of an eye gaze moving direction to the right or left indicated by arrows 85, 87, or by looking at the arrows positioned on the display 81, the alphabet is scrolled through. When the desired letter is displayed, the user can again look at the enter position 83 for a predetermined time duration so that the next letter can be added to the text at position 87. Such an inputting device will facilitate the communication for handicapped people. On the other hand, this kind of inputting device can also be used to control machines in harsh environments by simply analysing the eye gaze of the operator.

According to a variant to this embodiment, a complete keyboard could be illustrated in the display and using the eye gaze direction of a user who looks at a particular letter, this letter, which can be highlighted by a different colour, of the keyboard can be selected. To identify the selection a timer, an eye blink, a specific switch, the mouse button, a key of the keypad, can be used. One could also detect a mouth movement, e.g. the user shows its teeth, to confirm or choice. According to a further variant, double clicking by the eye can also be used to confirm the choice and the letter is introduced in an application, like a text processing software.

Figure 7C illustrates an additional application in which an operator is in front of a plurality of displays 91, 92 and 93 and an eye gaze detecting device 1, like the one illustrated in Figure 3, is used to identify the display a user is currently looking at. The user interface of the corresponding display can then be activated.

The inventive embodiments provide a simple and low cost possibility to detect eye gaze and to carry out input actions by analyzing the eye gaze direction or the moving direction of the eye gaze. By providing low cost but still reliable eye gaze direction analysis, the inventive eye gaze detecting devices 1 but also 51 can be used in a plurality of different application.

An eye gaze detecting device can be used in marketing applications to identify the eye gaze of a person looking at a given page (paper or computer screen) to determine whether advertising is indeed looked up or not. The device can be used in computers to activate windows or to wake up a computer. The invention can be used to survey an operator or a car driver to identify risks, such as sleep etc. It can be used as an interface tool for machinery, in particular in hazardous environments such as nuclear plants, biological or chemical experiments etc.. In computer applications, the eye gaze detection device can replace the mouse or even the keyboard which has advantageous effects when, for example, combined with privacy requirements, like with an automatic teller machine and, in military applications the invention can be used for automatic aiming purposes. The invention can also be used in medical applications, for example to determine the speed of eye movement in order to analyse neurological troubles of patients. This analysis only needs quick cameras (e.g. 200 images per second) and can replace other expensive systems based on Infrared helmets.

The various embodiments and variants of the invention can be combined in any combination.

**Claims**

1. A method for determining the position of an object in an image comprising the steps of:

    a) determining a first apparent position of the object in an image using a first detection method,
    b) determining a second apparent position of the object in an image using a second detection method,
    c) determining the difference between the first and second apparent position,
    d) deciding that the real position is based on the first apparent position in case the difference is less than a predetermined difference value and deciding that the real position is based on the second apparent position in case the difference is larger than the predetermined difference value.

2. Method according to claim 1, wherein the object is a part of a living body, in particular a face of a person.

3. Method according to claim 2, wherein the steps a) to d) are repeated for a sequence of subsequent images.

4. Method according to one of claims 1 to 3, wherein the first detection method provides the position with a lower error than the second detection method and/or wherein the second detection method has a lower drift than the first detection method.

5. Method according to one of claims 1 to 4, wherein the position of the object in step d) is determined by the first detection method, but the result of the second detection method is used to recalibrate the first detection method.

6. Method according to one of claims 1 to 5, wherein the first detection method is an optical flow method, in particular a Lucas-Kanade method, a block matching method or a Mean Shift method.

7. Method according to one of claims 1 to 6, wherein the second detection method is a face detect method, in particular based on a Haar algorithm, a segmentation algorithm or pixel colour based.

8. Method for controlling an input device, in particular the position of a cursor on a screen, using the method according to one of claims 1 to 7.

9. Method according to claim 8, wherein a change of position of the cursor from one image to the next is determined by the positional change of the object from the one image to the next image.

10. A method of determining an attitude or eye gaze direction of a person's face by

a) receiving image data of the person's face from a camera, the image data corresponding to the data of one, in particular only one, image of the person's head,
b) determining the position of at least three characteristic facial points in particular positioned away from the eyes of the person
c) determining the angular position of the face with respect to a reference system of the camera based on the positions of the characteristic facial points to thereby determine the attitude of the person's face.

11. Method according to claim 10, wherein only one camera captures the image, in particular from a fixed position independent of the person.

12. Method according to claim 10 or 11, further comprising an initialisation step of determining the distance between the person and a predetermined plane, e.g. the plane of the camera, in particular based on the real distance between two fixed facial points and their distance in the image or based on an estimated value, more in particular on an inter eye distance of 65mm +/-3 mm.

13. A method of determining the eye gaze direction comprising the method according to one of claims 10 to 12, wherein step b) comprises an additional sub-step of determining the position of at least one eye, in particular the iris of the eye, wherein the characteristic facial points are positioned away from the eye, and further comprising a step d) of determining the eye-gaze direction in the reference system of the camera based on the angular position of the face and the position of the at least one eye, in particular of the position of the iris and the position of the eyeball centre of the eye.

14. Attitude or Eye-gaze detecting device comprising one camera (3) and a processing unit (5) configured to carry out the method according to one of claims 1 to 13.

15. Computer program product, comprising one or more computer readable media having computer-executable instructions for performing the steps of the method according to one of claims 1 to 13.

104

face detect

102

optical flow

106 — compare between optical
flow and face detect

difference >
predicted
value?

NO

108

YES — 110

112 — recalibrate optical flow by
face detect

122 — position cursor on screen

100

FIG. 1a

FIG. 1b

FIG. 1c

Initializing - providing distance between user and camera  ~S1

↓

detect head/face in image  ~S2

↓

determine position of eyes and iris in image  ~S3

↓

determine position of additional characteristic facial points  ~S4

↓

determine angular position of face with respect to camera reference system  ~S5

↓

determine eye gaze direction in fixed camera reference system  ~S6

FIG. 2

FIG. 3

FIG. 4

FIG. 5

FIG. 6a

FIG. 6b

look up

FIG. 6c

look left

FIG. 6d

look right

FIG. 7a

FIG. 7b

FIG. 7c

**EUROPEAN SEARCH REPORT**

Application Number

EP 09 29 0444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ERIK MURPHY-CHUTORIAN ET AL: "HyHOPE: Hybrid Head Orientation and Position Estimation for vision-based driver head tracking" INTELLIGENT VEHICLES SYMPOSIUM, 2008 IEEE, IEEE, PISCATAWAY, NJ, USA, 4 June 2008 (2008-06-04), pages 512-517, XP031318965 ISBN: 978-1-4244-2568-6 * page 512, right-hand column, line 23 - page 513, right-hand column, line 30 * * page 515, left-hand column, paragraph IV - page 516, left-hand column, last line * * figure 2 * | 1-9,14, 15 | INV. G06T7/00 ADD. G06T7/20 |
| X | MASAKAZU MATSUGU ET AL: "Face Tracking Active Vision System with Saccadic and Smooth Pursuit" ROBOTICS AND BIOMIMETICS, 2006. ROBIO '06. IEEE INTERNATIONAL CON FERENCE ON, IEEE, PI, 1 December 2006 (2006-12-01), pages 1322-1328, XP031068978 ISBN: 978-1-4244-0570-1 * figures 1,2 * * page 1324, left-hand column, paragraph III.B - right-hand column * | 1-9,14, 15 | TECHNICAL FIELDS SEARCHED (IPC) G06T |

-/--

The present search report has been drawn up for all claims

| Place of search | Date of completion of the search | Examiner |
|---|---|---|
| Berlin | 18 December 2009 | Gauthier, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 09 29 0444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GERASIMOS POTAMIANOS ET AL: "A Joint System for Single-Person 2D-Face and 3D-Head Tracking in CHIL Seminars" MULTIMODAL TECHNOLOGIES FOR PERCEPTION OF HUMANS; [LECTURE NOTES IN COMPUTER SCIENCE;;LNCS], SPRINGER BERLIN HEIDELBERG, BERLIN, HEIDELBERG, vol. 4122, 6 April 2006 (2006-04-06), pages 105-118, XP019076842 ISBN: 978-3-540-69567-7 * figures 2,3 * * page 108, paragraph 2.1 * * page 112, paragraph 3.4 * | 1,14,15 | |
| A | MURIEL PRESSIGOUT ET AL: "Hybrid tracking approach using optical flow and pose estimation" IMAGE PROCESSING, 2008. ICIP 2008. 15TH IEEE INTERNATIONAL CONFERENCE ON, IEEE, PISCATAWAY, NJ, USA, 12 October 2008 (2008-10-12), pages 2720-2723, XP031374603 ISBN: 978-1-4244-1765-0 * page 2720, left-hand column, paragraph 1 - right-hand column * * page 2720, right-hand column, paragraph 2 - page 2721, right-hand column, paragraph 3.1 * * page 2722, right-hand column, line 6 - line 9 * | 1-7,14, 15 | |

TECHNICAL FIELDS
SEARCHED       (IPC)

-/--

The present search report has been drawn up for all claims

| Place of search | Date of completion of the search | Examiner |
|---|---|---|
| Berlin | 18 December 2009 | Gauthier, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 09 29 0444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WEI-KAI LIAO ET AL: "Integrating Multiple Visual Cues for Robust Real-Time 3D Face Tracking" ANALYSIS AND MODELING OF FACES AND GESTURES; [LECTURE NOTES IN COMPUTER SCIENCE], SPRINGER BERLIN HEIDELBERG, BERLIN, HEIDELBERG, vol. 4778, 20 October 2007 (2007-10-20), pages 109-123, XP019081573 ISBN: 978-3-540-75689-7 * page 110, line 13, paragraph 1. - page 114, paragraph 3.1 * ----- | 1-7,14, 15 | |
| A | MIKOLAJCZYK K ET AL: "Face detection and tracking in a video by propagating detection probabilities" IEEE TRANSACTIONS ON PATTERN ANALYSIS AND MACHINE INTELLIGENCE, IEEE SERVICE CENTER, LOS ALAMITOS, CA, US, vol. 25, no. 10, 1 October 2003 (2003-10-01), pages 1215-1228, XP011101847 ISSN: 0162-8828 * page 1215, left-hand column, paragraph 1. - right-hand column, last line * * page 1216, right-hand column, paragraph 2.3 - page 1217, left-hand column, paragraph 3.1 * * page 1219, left-hand column, paragraph 4.1 - page 1221, left-hand column, last line , paragraph 4.2 * ----- -/-- | 1-7,14, 15 | TECHNICAL FIELDS SEARCHED (IPC) |

The present search report has been drawn up for all claims

| Place of search | Date of completion of the search | Examiner |
|---|---|---|
| Berlin | 18 December 2009 | Gauthier, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

**Application Number**

EP 09 29 0444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHONG LUO ET AL: "Face tracking in video with hybrid of Lucas-Kanade and condensation algorithm" MULTIMEDIA AND EXPO, 2003. PROCEEDINGS. 2003 INTERNATIONAL CONFERENCE ON 6-9 JULY 2003, PISCATAWAY, NJ, USA,IEEE, vol. 3, 6 July 2003 (2003-07-06), pages 293-296, XP010651151 ISBN: 978-0-7803-7965-7 * pages III-293, left-hand column, paragraph 1. - right-hand column * * page III.294, left-hand column, paragraph 3. - page III.295, left-hand column, paragraph 3.2 * | 6 | |
| A | PALLEJA T ET AL: "Simple and robust implementation of a relative virtual mouse controlled by head movements" HUMAN SYSTEM INTERACTIONS, 2008 CONFERENCE ON, IEEE, PISCATAWAY, NJ, USA, 25 May 2008 (2008-05-25), pages 221-224, XP031293484 ISBN: 978-1-4244-1542-7 * page 222, left-hand column, paragraph III. - page 223, left-hand column, last line , paragraph III.B * * figures 1,2 * | 1,8,9 | TECHNICAL FIELDS SEARCHED (IPC) |

-----

-----

-/--

The present search report has been drawn up for all claims

| Place of search | Date of completion of the search | Examiner |
|---|---|---|
| Berlin | 18 December 2009 | Gauthier, J |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

.............................................................................................
& : member of the same patent family, corresponding
   document

EPO FORM 1503 03.82 (P04C01)

Europäisches
Patentamt

European
Patent Office

Office européen
des brevets

# EUROPEAN SEARCH REPORT

Application Number

EP 09 29 0444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YUN FU ET AL: "hMouse: Head Tracking Driven Virtual Computer Mouse" APPLICATIONS OF COMPUTER VISION, 2007. WACV '07. IEEE WORKSHOP ON, IEEE, PI, 1 February 2007 (2007-02-01), pages 1-6, XP031055139 ISBN: 978-0-7695-2794-9 * figures 1-3 * * page 2, left-hand column, paragraph 2 - page 3, right-hand column, last line , paragraph 4.4 * | 1,8,9 | |
| A | TU ET AL: "Face as mouse through visual face tracking" COMPUTER VISION AND IMAGE UNDERSTANDING, ACADEMIC PRESS, US, vol. 108, no. 1-2, 1 September 2007 (2007-09-01), pages 35-40, XP022227941 ISSN: 1077-3142 * figures 1,5,8-10 * * page 35, left-hand column, paragraph 1 - page 36, left-hand column, last line * * page 37, left-hand column, paragraph 3 - page 38, left-hand column, last line , paragraph 4 * | 1,8,9 | |
| X | YINGJIE PAN ET AL: "3-D Head Pose Estimation for Monocular Image" FUZZY SYSTEMS AND KNOWLEDGE DISCOVERY; [LECTURE NOTES IN COMPUTER SCIENCE;LECTURE NOTES IN ARTIFICIAL INTELLIGENCE;LNCS], SPRINGER-VERLAG, BERLIN/HEIDELBERG, vol. 3614, 26 July 2005 (2005-07-26), pages 293-301, XP019014662 ISBN: 978-3-540-28331-7 | 10-12 | TECHNICAL FIELDS SEARCHED (IPC) |
| Y | * page 293, paragraph 2 - page 300 * | 13 | |

-/--

The present search report has been drawn up for all claims

| Place of search | Date of completion of the search | Examiner |
|---|---|---|
| Berlin | 18 December 2009 | Gauthier, J |

EPO FORM 1503 03.82 (P04C01)

**Europäisches Patentamt**
**European Patent Office**
**Office européen des brevets**

# EUROPEAN SEARCH REPORT

Application Number

EP 09 29 0444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/22872 A (MICROSOFT CORP [US]) 5 April 2001 (2001-04-05) * page 4, line 11 - line 25 * * page 7, line 24 - line 29 * * page 10, line 29 - page 11, line 25 * * page 14, line 11 - page 15, line 19 * * figures 2,4,6,7 * ----- | 10-12 | |
| X | US 2008/187175 A1 (KIM JUNG-BAE [KR] ET AL) 7 August 2008 (2008-08-07) * figures 2-4,6 * * paragraph [0037] - paragraph [0048] * ----- | 10-12 | |
| Y | WANG J-G ET AL: "Estimating the eye gaze from one eye" COMPUTER VISION AND IMAGE UNDERSTANDING, ACADEMIC PRESS, US, vol. 98, no. 1, 1 April 2005 (2005-04-01), pages 83-103, XP004665543 ISSN: 1077-3142 * page 84, line 1, paragraph 1 - page 85, last line * * page 93, line 1, paragraph 4.1.3 - page 94, last line * * figures 1-3,6 * ----- | 13 | TECHNICAL FIELDS SEARCHED (IPC) |
| Y | WO 02/09025 A (SEEING MACHINES PTY LTD [AU]; EDWARDS TIMOTHY [AU]; HEINZMANN JOCHEN []) 31 January 2002 (2002-01-31) * page 12, line 24 - page 14, line 21 * * page 15, line 14 - line 30 * * page 17, line 13 - line 19 * * page 19, line 3 - line 16 * * figures 1,2,5,6 * ----- -/-- | 13 | |

The present search report has been drawn up for all claims

| Place of search | Date of completion of the search | Examiner |
|---|---|---|
| Berlin | 18 December 2009 | Gauthier, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 2 261 857 A1**

Europäisches
Patentamt

European
Patent Office

Office européen
des brevets

# EUROPEAN SEARCH REPORT

Application Number

EP 09 29 0444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | STYLIANOS ASTERIADIS ET AL: "Estimation of behavioral user state based on eye gaze and head poseâ application in an e-learning environment"<br>MULTIMEDIA TOOLS AND APPLICATIONS, KLUWER ACADEMIC PUBLISHERS, BO,<br>vol. 41, no. 3,<br>25 October 2008 (2008-10-25), pages 469-493, XP019684907<br>ISSN: 1573-7721<br>* page 472, line 12 - line 16 *<br>* page 473, line 1, paragraph 1.3 - line 20 *<br>* page 475, line 1, paragraph 2.1 - page 476, line 40 *<br>* page 478, line 1 - line 10 *<br>* page 479, line 6 - line 8 *<br>* figures 1,3,4 * | 12 | |
| A | HEINZMANN J ET AL: "3-D facial pose and gaze point estimation using a robust real-time tracking paradigm"<br>AUTOMATIC FACE AND GESTURE RECOGNITION, 1998. PROCEEDINGS. THIRD IEEE INTERNATIONAL CONFERENCE ON NARA, JAPAN 14-16 APRIL 1998, LOS ALAMITOS, CA, USA,IEEE COMPUT. SOC, US,<br>14 April 1998 (1998-04-14), pages 142-147, XP010277652<br>ISBN: 978-0-8186-8344-2<br>* figures 1-3 *<br>* page 143, right-hand column, paragraph 3 - page 144, left-hand column *<br>* page 145, left-hand column, paragraph 5 - page 146, left-hand column *<br>* page 146, right-hand column, paragraph 7 - page 147, left-hand column *<br>-/-- | 13 | TECHNICAL FIELDS SEARCHED (IPC) |

The present search report has been drawn up for all claims

| Place of search | Date of completion of the search | Examiner |
|---|---|---|
| Berlin | 18 December 2009 | Gauthier, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

26

**EUROPEAN SEARCH REPORT**

Application Number

EP 09 29 0444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | XUNHENG WANG ET AL: "Fast Face Orientation Estimation from an Uncalibrated Monocular Camera" IMAGE AND SIGNAL PROCESSING, 2008. CISP '08. CONGRESS ON, IEEE, PISCATAWAY, NJ, USA, 27 May 2008 (2008-05-27), pages 186-190, XP031287019 ISBN: 978-0-7695-3119-9 * page 186, left-hand column, line 10, paragraph 1. - right-hand column, line 24 * * page 187, left-hand column, paragraph 2.2 - right-hand column * * right-hand column, line 1, paragraph 2.3 - last line * | 10-12 | |
| A | GEE A ET AL: "Determining the gaze of faces in images" IMAGE AND VISION COMPUTING, ELSEVIER, GUILDFORD, GB, vol. 12, no. 10, 1 December 1994 (1994-12-01), pages 639-647, XP026655929 ISSN: 0262-8856 [retrieved on 1994-12-01] * page 639, right-hand column, line 35 - page 641, left-hand column, last line * | 10-13 | TECHNICAL FIELDS SEARCHED (IPC) |
| A | DEMENTHON D F ET AL: "Model-based object pose in 25 lines of code" INTERNATIONAL JOURNAL OF COMPUTER VISION, KLUWER ACADEMIC PUBLISHERS, NORWELL, US, vol. 15, no. 1, 1 January 1995 (1995-01-01), pages 123-141, XP002513566 ISSN: 0920-5691 * page 1 * | 10-13 | |

The present search report has been drawn up for all claims

| Place of search | Date of completion of the search | Examiner |
|---|---|---|
| Berlin | 18 December 2009 | Gauthier, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

Europäisches
Patentamt
European
Patent Office
Office européen
des brevets

Application Number

EP 09 29 0444

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION
SHEET B**

Application Number

EP 09 29 0444

The Search Division considers that the present European patent application does not comply with the
requirements of unity of invention and relates to several inventions or groups of inventions, namely:

    1. claims: 1-9,14,15

        detection and correction of tracking drift in a head tracker

    1.1. claims: 8,9

        Vision-based and contact-less remote control of a cursor on
        a screen.
            ---

    2. claims: 10-13

        Determination of the pose of the head in a head tracker
            ---

Please note that all inventions mentioned under item 1, although not
necessarily linked by a common inventive concept, could be searched
without effort justifying an additional fee.

**ANNEX TO THE EUROPEAN SEARCH REPORT
ON EUROPEAN PATENT APPLICATION NO.**

EP 09 29 0444

This annex lists the patent family members relating to the patent documents cited in the above-mentioned European search report.
The members are as contained in the European Patent Office EDP file on
The European Patent Office is in no way liable for these particulars which are merely given for the purpose of information.

18-12-2009

| Patent document cited in search report | | Publication date | Patent family member(s) | | Publication date |
|---|---|---|---|---|---|
| WO 0122872 | A | 05-04-2001 | AU<br>US | 7841500 A<br>6741756 B1 | 30-04-2001<br>25-05-2004 |
| US 2008187175 | A1 | 07-08-2008 | KR | 20080073933 A | 12-08-2008 |
| WO 0209025 | A | 31-01-2002 | EP<br>JP<br>US | 1320830 A1<br>2004504684 T<br>2003169907 A1 | 25-06-2003<br>12-02-2004<br>11-09-2003 |

For more details about this annex : see Official Journal of the European Patent Office, No. 12/82

EPO FORM P0459